# EUROPÄISCHE PATENTSCHRIFT

(11) **EP 1 408 898 B1**
(45) Veröffentlichungstag und Bekanntmachung des Hinweises auf die Patenterteilung: **01.12.2004**
(21) Anmeldenummer: 02764780.9
(22) Anmeldetag: 25.07.2002
(51) Int. Cl.: A61F 7/00

(54) **THERMISCHER APPLIKATOR UND APPLIKATORSYSTEM**
THERMAL APPLICATOR AND APPLICATION SYSTEM
APPLICATEUR ET SYSTEME D'APPLICATEUR THERMIQUES

(30) Priorität: 25.07.2001 CH 139001
(43) Veröffentlichungstag der Anmeldung: 21.04.2004
(73) Patentinhaber: ZIMMER ELEKTROMEDIZIN GmbH, D-89231 Neu-Ulm (DE)
(72) Erfinder: BOEGELEIN, Konrad, 89231 Neu-Ulm (DE); TÖPFER, Jörg, 07749 Jena (DE)
(74) Vertreter: Hasler, Erich, Dr.
(86) Internationale Anmeldenummer: PCT/EP2002/008302
(87) Internationale Veröffentlichungsnummer: WO 2003/009788

(56) Entgegenhaltungen:
- WO-A-96/12454
- US-A- 4 660 388
- US-A- 5 392 847
- US-A- 5 443 488
- US-B1- 6 171 333

## Beschreibung

Die Erfindung betrifft einen Applikator und ein Applikatorsystem für die Erzeugung und Applikation eines kalten und/oder warmen gasförmigen Medienstromes auf eine zu behandelnde Körperstelle oder einen zu behandelnden Körper.

Im Altertum war bereits bekannt, dass Schmerzen durch Behandlung mit Kälte gelindert werden können (sog. Kryotherapie). In der heutigen Zeit wird die Kaltlufttherapie nicht nur zur Behandlung von Schmerzen, sondern beispielsweise auch zur Behandlung von akuten oder chronischen Erkrankungen des menschlichen Stütz- und Bewegungsapparates eingesetzt.

Bekannt sind u.a. Geräte für die Kaltlufttherapie, mit welchen Umgebungsluft angesaugt, abgekühlt und über einen Schlauch wieder abgegeben wird. Nachteilig an diesen Geräten ist, dass die Öffnung des Schlauches manuell über die zu behandelnde Körperstelle oder -areal geführt werden muss. Entsprechend kann der Abstand der Schlauchöffnung zur zu behandelnden Körperstelle, die Verteilung der abgegebenen Luftmenge und der Luftauftreffwinkel und die thermodynamische Wirkung stark varüeren. Dies bedeutet, dass eine Überwachung, Quantifizierbarkeit, Differenzierbarkeit und Kontrolle der Applikation und des Applikationsortes schlecht möglich ist. Ein weiterer Nachteil ist, dass das Körperteil auf der Unterlage aufliegt und daher zirkulär nicht erreicht werden kann. Nachteilig ist auch, dass für die Dauer der Therapie entsprechendes Personal nötig ist.

Die US 5,214,860 offenbart eine Gips-Trocknungseinrichtung, welche in Verbindung mit Druckluft verwendet wird. Die Gips-Trocknungseinrichtung besitzt einen Balg mit einer Innen- und einer Aussenwand, welche miteinander verschweisst sind und eine Expansionskammer definieren. An der Expansionskammer sind eine oder mehrere Lufteintrittsöffnungen für die Zufuhr von Luft in die Expansionskammer vorgesehen. Weiter sind in der Innenwand eine Vielzahl von Luftaustrittsöffnungen vorgesehen. Die US 5,214,860 schlägt weiter vor, einen im wesentlichen flachen Balg zu verwenden und diesen so an den Gips zu legen, dass ein gleichmässiger Luftstrom in verschiedene Teile des Gipses eindringen kann.

Die DE-OS-34 14 094 bezieht sich auf eine Anlage zur Behandlung von thermischen Verletzungen. Die Anlage umfasst ein Gebläse, ein Filtersystem, einen Elektroheizer und eine an diesen angeschlossene Steuereinheit mit einem Temperaturgeber sowie eine Einrichtung zum Fördern der abakteriellen Luft in eine Behandlungskammer. Die Behandlungskammer ist ein transparenter, durch den Luftstrom elastisch aufblasbarer Sack.

Die US 5,938,693 offenbart ein therapeutisches System mit einem Generator zur Erzeugung eines erwärmten und feuchten Luftstroms zur Gesichtsbehandlung. Der Generator hat ein Gehäuse mit einer Dampfkammer und einem Flüssigkeitsreservoir. Durch Heizmittel kann das im Flüssigkeitsreservoir vorhandene Fluid aufgeheizt werden, wobei der dabei entstehende Dampf in der Dampfkammer aufgenommen wird. Ein Interface, welches mit dem Generator in Verbindung steht, erlaubt es, die feuchte und erwärmte Luft auf eine gewünschte Behandlungsfläche zu lenken. Das Interface hat vorzugsweise die Gestalt einer Maske mit einer inneren und einer äusseren Auskleidung. Die innere Auskleidung besteht aus einem atmungsaktiven Textil, z.B. einer mikroporösen Membran, mit einer bestimmten ersten Permeabilität. Die äussere Auskleidung hat eine bestimmte zweite Permeabilität, welche geringer ist als die Permeabilität der inneren Wand. Des weiteren kann das Interface die Gestalt eines Kissens aufweisen, welches mittels am Kissen vorgesehenen Bändern an bestimmten Körperstellen fixierbar ist.

Das Interface der US 5,938,693 ist einfach und zweckmässig. Allerdings ist es schwierig, während des Betriebs über die ganze Fläche des Interface eine gleichmässige Temperatur aufrechtzuerhalten, da die erwärmte Luft nur an einer Stelle zugeführt wird. Die US 5,938,693 schlägt deshalb vor, in der inneren Auskleidung geeignete Kanäle vorzusehen, um die feuchte Luft in gewünschter Weise umzulenken. Weiter besteht beim Interface der US 5,938,693 die Gefahr, dass die innere und äussere Auskleidung aneinandergepresst werden, sodass an diesen Stellen jeglicher Gasfluss unterbrochen ist. Dies hat zur Folge, dass an diesen Stellen praktisch kein Gas austreten kann.

US-B1-6 171 333 offenbart einen Applikator gemäß Oberbegriff von Anspruch 1.

Es ist daher Aufgabe der vorliegenden Erfindung, eine Vorrichtung vorzuschlagen, mit welcher diese Nachteile weitgehend vermieden werden können. Es ist insbesondere ein Ziel, einen Applikator resp. ein Applikatorsystem bereitzustellen, mittels welchem eine bessere Kontrolle und Überwachung, Quantifizierbarkeit, Differenzierbarkeit sowie Reproduzierbarkeit der Applikation und des Applikationsortes möglich ist. Ein weiteres Ziel ist es, weitere Verwendungen der Kaltlufttherapie bzw. "Wechselluftanwendung" vorzuschlagen. Noch ein Ziel ist es, eine möglichst gleichmässige Verteilung und Temperatur des zugeführten Medienstromes zu erreichen.

### Beschreibung

Erfindungsgemäss ist ein Applikator gemäss Oberbegriff von Anspruch 1 dadurch gekennzeichnet, dass das Gasdurchtrittsmittel ein dreidimensionales, gasdurchlässiges aus Fasern oder Fäden gebildetes Abstandsgewebe oder Abstandsgewirke einer ersten Art ist, welches eine Vielzahl von nach allen Seiten orientierten Gasdurchtrittsöffnungen oder Gasdurchtrittskanälen definiert, sodass im Betrieb eine Gasverteilung sowohl parallel als auch senkrecht zur Gasdurchtrittsfläche bewirkt ist.. Das Gasdurchtrittsmittel bewirkt bei der Verwendung eine gleichmässige Verteilung des gasförmigen Mediums im Bereich der Gasdurchtrittsfläche, bevor das Medium durch die Durchtrittsöffnungen der Gasdurchtrittsseite auf die zu behandelnde Körperstelle gelangt. Die dreidimensionale Struktur ist ein dreidimensionales Abstandsgewebe oder -gewirke. Der Vorteil Gewebes oder Gewirkes ist deren luftführende und luftverteilende Funktion. Dadurch ist eine hohe Vergleichmässigung der Temperaturverteilung im Gasmedium erreichbar. Die dreidimensionale Struktur bewirkt im Betrieb eine Gasverteilung sowohl parallel als auch senkrecht zur Gasdruchtrittsfläche. Der Gasaustritt aus der Struktur kann nach allen Richtungen erfolgen, erfolgt jedoch vorzugsweise zur körperzugewandten Seite des Applikators. Wenn das dreidimensionale Abstandsgewebe oder -gewirke aus einem Feuchtigkeit aufnehmenden und/ oder ableitenden Material hergestellt ist, so kann das Material auch ein richtungsorientiert einsetzbares Feuchteleitvermögen bieten. Dies erweist sich insbesondere bei Wechseltemperaturenanwendungen als vorteilhaft. Als Materialien eignen sich Fasern mit feuchtigkeitsauf- resp. abgebenden Eigenschaften wie z.B. Baumwoll oder Viskosefasern.

Zweckmässigerweise definiert die Einrichtung zum Zuführen eines Gases mindestens einen Verteilraum, welcher eine Vielzahl von Austrittsöffnungen hat. Der Verteilraum kann als ein Schlauch, ein Rohr, ein Sack oder dergleichen ausgebildet sein. Vorzugsweise erstreckt sich der wenigstens eine Verteilraum im Bereich des flächenhaft ausgebildeten Gasdurchtrittsmittels. Dies hat den Vorteil, dass das Gas, in laminarer Strömung über die Applikatorfläche verteilt, zugeführt werden kann. Gemäss einer bevorzugten Ausführungsform sind eine Mehrzahl von in Abstand voneinander angeordneten Gasverteilräumen vorgesehen.

Der wenigstens eine Verteilraum hat eine Vielzahl von Austrittsöffnungen. Durch die Vielzahl von Austrittsöffnungen wird eine Grobverteilung des in den Applikator eingeführten Gases bewirkt, sodass weniger die Gefahr besteht, dass sich über die Applikationsfläche ein Temperaturgradient ausbildet. Obwohl die Austrittsöffnungen über den gesamten Umfang des Verteilraums verteilt sein können, sind gemäss einer bevorzugten Ausführungsform die Austrittsöffnungen nur entlang eines länglichen Umfangabschnittes vorgesehen. Der Verteilraum kann dann so im oder am dreidimensionalen Gewebe angeordnet sein, dass die Austrittsöffnungen wenigsten teilweise in Richtung zur Gasdurchtrittsseite orientiert sind.

Vorteilhaft ist der Applikator flexibel ausgebildet ist, sodass der Applikator an unterschiedliche Körperpartien anpassbar resp. anlegbar ist. Die Gaszuführungseinrichtung, z.B. ein oder mehrere Schläuche oder Kissen, kann in oder an der dreidimensionalen Struktur angeordnet sein. Es sind also Ausführungsformen denkbar, bei welchen Gasverteil-Schläuche oder -Kammern direkt in der dreidimensionalen Struktur integriert sind.

In einer zweckmässigen Ausführungsform hat der Applikator eine zweite, gasundurchlässige Seite, welche der ersten Gasdurchtrittsseite gegenüberliegt, und die Verteilräume sind zwischen der ersten und zweiten Seite angeordnet. Ein solcher Applikator hat den Vorteil, dass das Gas im wesentlichen nur durch die Gasdurchtrittsseite austreten kann. Der Applikator kann sowohl ein- als auch mehrlagig ausgebildet sein. Vorzugsweise sind die Austrittsöffnungen der Verteilräume in Abstand zur Gasdurchtrittsseite der dreidimensionalen Struktur angeordnet. Dies hat den Vorteil, dass eine gute Feinverteilung des durch die Austrittsöffnungen strömenden Mediums erreichbar ist. Des weiteren können am Verteilraum Leitflächen für die Direktion des während des Betriebs austretenden Gasmediums in Richtung zur Gasdurchtrittsseite vorgesehen sein.

Vorteilhaft sind am Applikator Befestigungsmittel zum Festmachen des Applikators an oder über der zu behandelnden Körperpartie vorgesehen. Die Befestigungsmittel können z.B. ein Band, eine Gurte oder dergleichen und integral mit dem Applikator sein. Zweckmässigerweise besitzen die Befestigungsmittel ein Klettenverschluss oder Ähnliches, um den Applikator an einer Körperstelle befestigen zu können.

Der Applikator kann in verschiedenen Ausführungen, z.B. als Kissen oder Manschette, bereitgestellt werden. Der Applikator kann mindestens ein Fenster aufweisen, durch welches beispielsweise eine Behandlung mittels eines Laserstrahles möglich ist. Die Lichtbehandlung kann gleichzeitig oder zeitlich versetzt zur Kalt/Warmluftbehandlung erfolgen. Das Fenster kann eine Ausnehmung in der dreidimensionalen Struktur sein, welche beispielsweise durch einen transparenten Kunststoff oder Glas abgedeckt ist. An den Applikator kann eine oder mehrere Verbindungsleitungen angeschlossen sein, welche mit einem Kälte- und/oder Wärmegerät in Verbindung stehen.

Gegenstand der vorliegenden Erfindung ist auch ein Applikatorsystem mit wenigstens einem Kälte- und/ oder Wärmegerät zur Erzeugung eines gekühlten oder erwärmten gasförmigen Mediums und einem Applikator gemäss einem der Ansprüche 1 bis 18 sowie gegebenenfalls Verbindungsmittel zum Verbinden des Kälte-/Wärmegeräts mit dem Applikator. Eine bevorzugte Ausführungsform sieht vor, dass das Applikatorsystem einen Applikator gemäss einem der Ansprüche 1 bis 18 sowie ein Kältegerät zur Erzeugung eines gasförmigen Medienstromes mit einer Temperatur unterhalb der üblichen Umgebungstemperatur und insbesondere tiefer als 10 °C besitzt.

Der Applikator resp. das Applikatorsystem ist einsetzbar für die kontrollierte und gezielte - flächen und/oder volumenbegrenzbaren Verteilung und/oder Dosierung von Kaltluft oder Kälte und/oder Warmluft oder Wärme - bei allen Erkrankungen und Zuständen, die direkt und indirekt mit Spastik, Ödemen, zirkulatorischen Problemen sowie Veränderungen von Spannungs, Längen-, Struktur-, biomechanischen-, neurogenen, stoffwechselmässigen und viskösen Charakteristika im zellulären, geweblichen, organischen, genetischen und physiologischen Bereich, insbesondere der Muskulatur, der Sehnen, der Knochen, des Gefäss- und Nervensystems von/bei Lebewesen zu tun haben. Vorteilhaft kann das Applikatorsystems zur Behandlung von zirkulatorischen Problemen im Herz-Kreislauf-System eingesetzt werden. Eine weitere Verwendung betrifft den Einsatz des erfindungsgemässen Applikators zur Effektivierung und Leistungssteigerung im muskulären und neuromuskulären Bereich und Atmung. Auch die Verwendung des Applikators zur positiven Stimulation des Immunsystems und des psychovegetativen Systems ist denkbar.

Die Anwendung führt zu einer Effektivierung und Leistungssteigerung des neuromuskulären Systems und des Gefässsystems, sowie zu einer günstigen Beeinflussung und/ oder Verbesserung der biomechanischen, neurophysiologischen und stoffwechselmässigen Qualitäten der Muskulatur und Sehnen, des gesamten Stütz- und Bewegungsapparates einschliesslich aller sonstigen Gewebsstrukturen. Es werden hormonelle, endokrine, zirkulatorische und mikrozirkulatorischen Aspekte des Körpers bezw. Körperareals günstig beeinflusst. Auch werden die Kriterien zur Verhinderung bezw. Beseitigung von Ödemen positiv beeinflusst. Es fördert hämodynamische, visköse und zirkulatorische Aspekte der Körperflüssigkeiten.

Nachfolgend wird die Erfindung unter Bezugnahme auf die Figuren beispielhaft näher erläutert. Es zeigt:
- Fig. 1a: im Querschnitt ein erstes Ausführungsbeispiel eines erfindungsgemässen Applikators mit in einer dreidimensionalen, gasdurchlässigen Struktur aufgenommenen Schläuchen;
- Fig. 1b: einen Längsschnitt durch das erste Ausführungsbeispiel von Fig. 1a entlang der Linie 1b-1b;
- Fig. 1c: einen Schnitt durch das erste Ausführungsbeispiel von Fig. 1a entlang der Linie 1c-1c, sowie Vorder- und Rückansichten des Applikators;
- Fig. 2a: im Querschnitt ein zweites Ausführungsbeispiel eines Applikators mit einer gasundurchlässigen Oberseite;
- Fig. 2b: einen Längsschnitt durch das zweite Ausführungsbeispiel von Fig. 2a entlang der Linie 2b-2b;
- Fig. 3a: im Querschnitt ein drittes Ausführungsbeispiel eines Applikators mit einer aus mehreren Lagen bestehenden dreidimensionalen Struktur;
- Fig. 3b: einen Längsschnitt durch das dritte Ausführungsbeispiel von Fig. 3a entlang der Linie 3b-3b;
- Fig. 3c: einen Schnitt durch das dritte Ausführungsbeispiel von Fig. 3a entlang der Linie 3c-3c, sowie Vorder- und Rückansichten des Applikators;
- Fig. 4a: ein viertes Ausführungsbeispiel mit auf der dreidimensionalen Struktur angeordneten Gas-Verteilschläuchen im Querschnitt;
- Fig. 4b: einen Längsschnitt durch das vierte Ausführungsbeispiel entlang der Linie 4b-4b;
- Fig. 5a: ein fünftes Ausführungsbeispiel eines Applikators in Gestalt einer faltbaren Tasche im Querschnitt;
- Fig. 5b: einen Mittelschnitt durch das Ausführungsbeispiel von Fig. 5a;
- Fig. 6: die Verwendung eines kissenförmigen Applikators an der Schulter eines Patienten;
- Fig. 7: einen manschettenartigen Applikator, welcher über den Unterarm eines Patienten gestülpt ist;
- Fig. 8: ein sechsten Ausführungsbeispiel eines kissenförmigen Applikators mit in einem dreidimensionalen Fasergebilde integrierten Verteilschläuchen;
- Fig. 9: in stark vergrössertem Massstab einen Ausschnitt aus dem dreidimensionalen Fasergebilde des Ausführungsbeispiels von Fig. 8;
- Fig. 10: schematisch einen rohrförmigen Applikator mit einem Bürstenansatz zur Verteilung des gasförmigen Mediums.

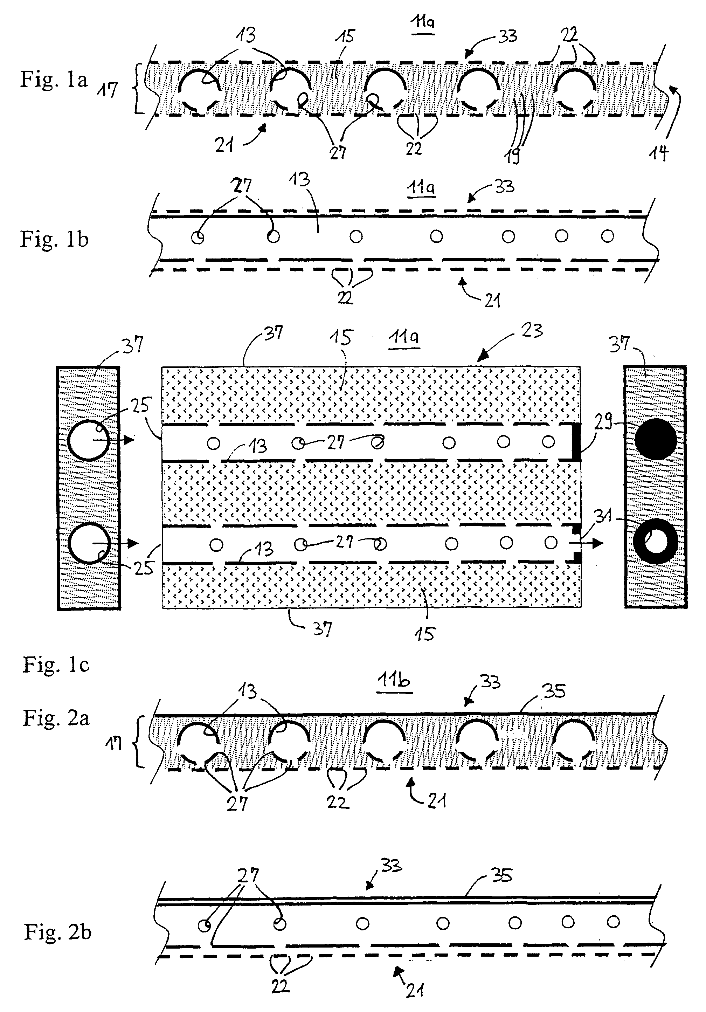

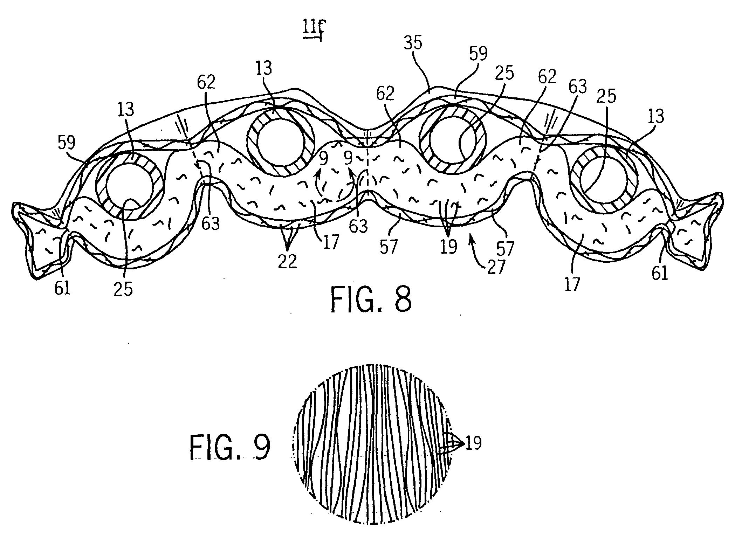

Das erste Ausführungsbeispiel eines Applikators 11a gemäss Figur 1 besitzt mehrere in Abstand voneinander angeordnete Gaszuführungsmittel in Gestalt von Schläuchen 13, welche mit Gasverteilmittel 14 zusammenwirken. Die Gasverteilmittel 14 besitzen eine gasdurchlässige dreidimensionale Struktur 15. In dieser dreidimensionalen Struktur 15 sind die Schläuche 13 aufgenommen. Die dreidimensionale Struktur 15 ist als flächiges, textiles Abstandsgewebe oder Abstandsgewirke ausgebildet. Das Abstandsgewirke hat eine Lage 17, z.B. ein Vlies oder Filz, die nach allen Seiten gasdurchlässig ist. Gemäss einer bevorzugten Ausführungsform ist die Lage 17 aus Fasern oder Fäden 19 gebildet. Die Fasern oder Fäden 19 definieren eine Vielzahl von nach allen Seiten orientierten Gasdurchtrittsöffnungen oder -kanälen, welche im Betrieb eine Feinverteilung des aus den Schläuchen austretenden Gases bewirken. Die bei der Anwendung zum Körper orientierte Seite der dreidimensionalen Struktur 15 definiert eine Gasdurchtrittsseite 21 (nachfolgend auch Unterseite oder Anwendungsseite des Applikators genannt). Wenigstens die Gasdurchtrittsseite 21 kann aus einem Gewebe oder Gewirke einer zweiten Art bestehen, mit welchem das dreidimensionale Abstandsgewebe oder Abstandsgewirke verbunden ist (s. Fig. 8 und Beschreibung dazu). Die Gasdurchtrittsseite 21 ist durch eine Vielzahl von Gasdurchtrittsöffnungen 22 charakterisiert.

Der Applikator 11a ist beispielsweise als rechteckiges Kissen 23 ausgebildet. Die Schläuche 13 sind in Abstand voneinander angeordnet in der dreidimensionalen Struktur 15 aufgenommen. Die Schläuche 13 besitzen mindesten je eine Eintrittsöffnung 25 und eine Mehrzahl von Austrittsöffnungen 27. Die Austrittsöffnungen 27 können über den gesamten Umfang des Schlauches 13 verteilt sein. Denkbar ist, die Austrittsöffnungen 27 nur entlang eines Längsabschnittes vorzusehen und die Austrittsöffnungen 27 senkrecht oder in einem spitzen Winkel zur Durchtrittsseite 21 zu orientieren. Die Austrittsöffnungen 27 können in gleichmässigen oder ungleichmässigen Abstand voneinander angeordnet sein. Vorzugsweise nimmt der Abstand der Austrittsöffnungen 27 voneinander mit der Entfernung von der Gaseintrittsöffnung 25 ab, d.h. in Abhängigkeit von der Entfernung von der Gaseintrittsöffnung nimmt die Austrittsfläche pro Längeneinheit zu. In Experimenten hat sich nämlich gezeigt, dass dies zu einer Vergleichmässigung des Gasvolumenstromes und der Temperaturverteilung beiträgt. Das entfernte Ende des Schlauches 13 kann mit einem Stopfen 29 verschlossen sein. Denkbar ist, anstelle eines Stopfens 29 ein regulierbares Ventil 31 vorzusehen, damit der Gasvolumenstrom einstellbar ist.

Das Ausführungsbeispiel 11b (Figur 2) unterscheidet sich vom ersten dadurch, dass die zur Gasdurchtrittsseite 21 gegenüberliegende, im Betrieb körperabgewandte Seite 33 des Applikators 11b (nachfolgend auch Rückseite des Applikators genannt) eine gasundurchlässige Deckschicht 35 ist. Denkbar ist, auch die Seitenkanten 37 mit einer gasundurchlässigen Deckschicht zu versehen, sodass in die Schläuche 13 eintretendes Gas nur durch die Durchtrittsseite 21 entweichen kann.

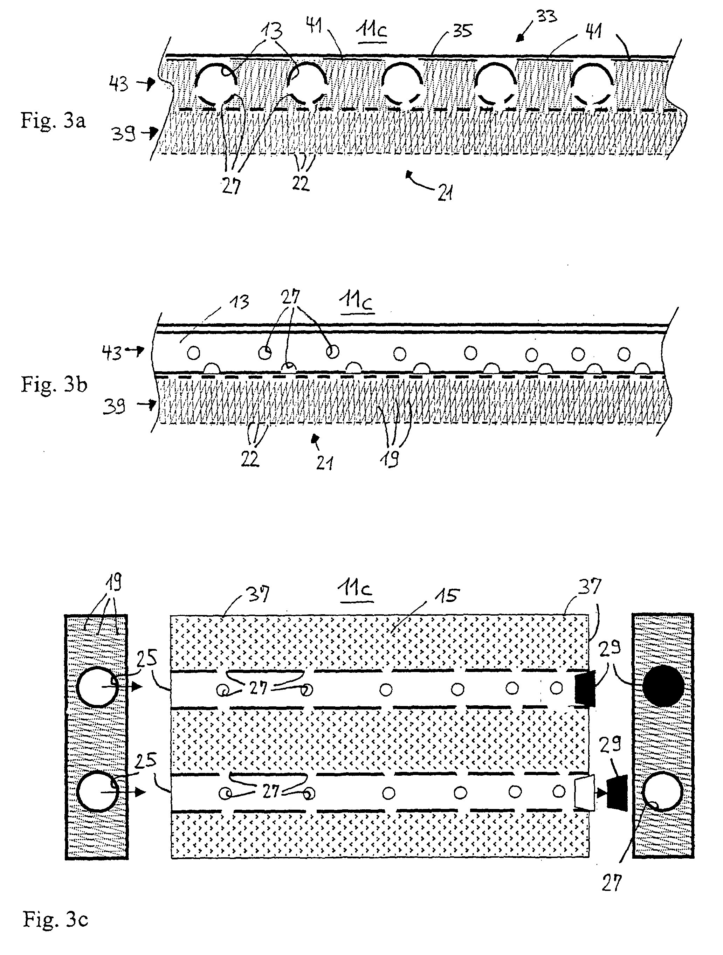

Das Ausführungsbeispiel 11c gemäss Figur 3 zeichnet sich dadurch aus, dass die dreidimensionale Struktur mehrlagig ist. Sie besitzt eine durchgehende untere Lage 39, auf welcher Schläuche 13 in Abstand voneinander angeordnet sind. Die untere Lage 39 ist vorzugsweise ein dreidimensionales Abstandsgewebe oder -gewirke 15, wie bereits oben beschrieben. Zwischen den Schläuchen 13 sind Bahnen 41 eines weiteren Abstandsmaterials vorgesehen. Die Bahnen 41 können aus dem gleichen Material wie die untere Lage 39 bestehen. Schläuche 13 und die dazwischen angeordneten Bahnen 41 definieren eine obere Lage 43. Auf der oberen Lage 43 kann eine gasundurchlässige Deckschicht 35 wie beim zweiten Ausführungsbeispiel angeordnet sein. Deutlich erkennbar ist, dass die Austrittsöffnungen 27 in Richtung zum Schlauchende hin einen immer kleineren Abstand voneinander aufweisen (Fig. 3b und 3c).

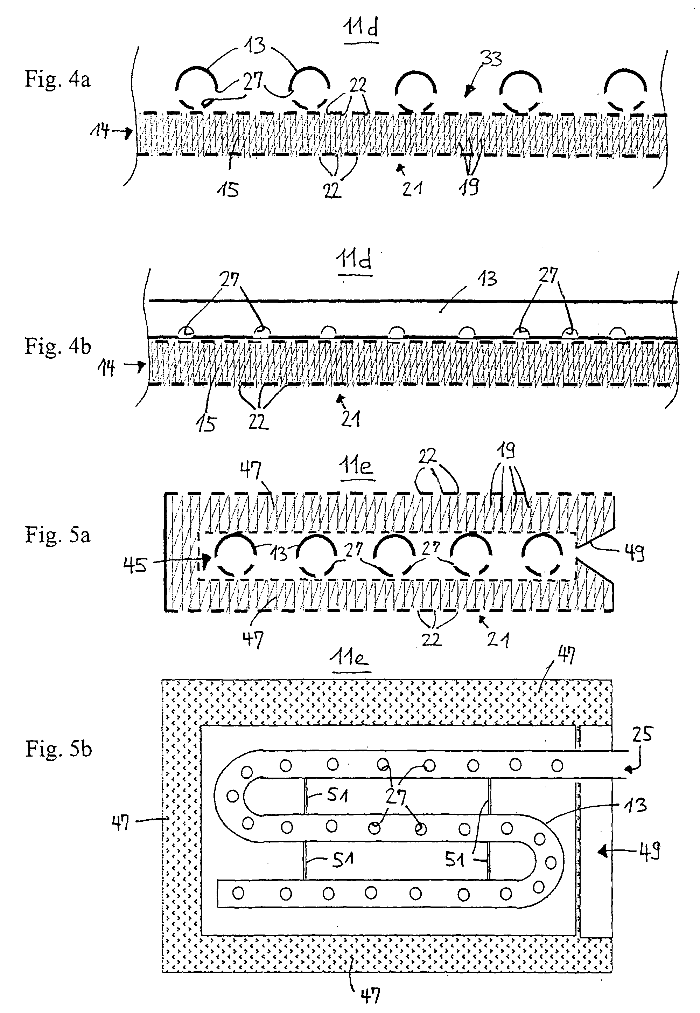

Ausführungsbeispiel 11d (Figur 4) unterscheidet sich von demjenigen von Figur 3 dadurch, dass die Schläuche 13 auf der aus einem dreidimensionalen Abstandsgewebe oder -gewirke bestehenden Struktur 15 in Abstand voneinander angeordnet sind und keine weiteren Lagen oder eine gasundurchlässige Deckschicht vorgesehen ist. Austrittsöffnungen 27 sind lediglich an der unteren Rohrhälfte der Schläuche 13 vorgesehen. Die Austrittsöffnungen 27 sind in einem Winkel zur Oberseite des Abstandsgewebe oder -gewirke orientiert. Die Schläuche 13 können durch nicht näher gezeigte lösbare oder nicht lösbare Befestigungsmittel an der Oberseite des dreidimensionalen Abstandsgewebes oder -gewirke angeordnet sein.

Figur 5 zeigt ein weiteres mehrlagiges Ausführungsbeispiel 11e, bei welchem die Gaszuführungsmittel 13 eine innere Lage 45 definieren, welche sandwichartig von zwei Lagen 47 eines dreidimensionalen textilen Abstandsgewebe oder -gewirke umgeben ist. Der Applikator 11e kann als aufklappbare Tasche mit einer Öffnung 49 ausgebildet sein. Dadurch ist ein Austausch des Innenlebens des Applikators jederzeit möglich. Die Schläuche 13 können einstückig und in der inneren Lage 45 mäanderartig verlegt sein (Figur 5b). Distanzhalter 51 fixieren die gegenseitige Lage der Schläuche.

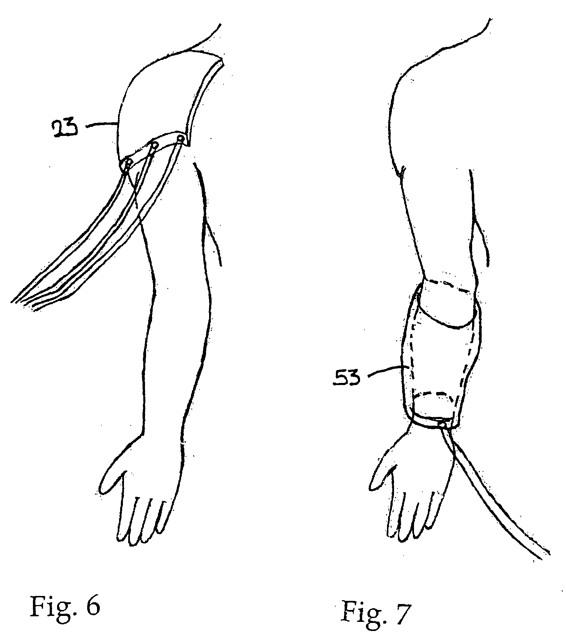

Die Figuren 6 und 7 zeigen konkrete Ausführungs- und Anwendungsbeispiele des erfindungsgemässen Applikators. In Figur 6 ist ein als Kissen 23 ausgebildeter Applikator 11 über die Schulter einer Person gelegt. Durch die Schläuche 13 kann ein kaltes oder erwärmtes gasförmiges Medium an die betreffende Körperstelle appliziert werden.

Figur 7 zeigt einen Applikator in Gestalt einer Manschette 53, welche über den Unterarm einer zu behandelnden Person gestülpt ist.

Der Applikator 11f gemäss Figur 8 besitzt eine aus Fasern bestehende, der Gasfeinverteilung dienende Lage 17, welche an einem durchlässigen Gewebe oder Gewirke 57 angeordnet ist. Das Gewebe oder Gewirke 57 ist grobmaschig und bildet die Gasdurchtrittsseite 21 mit Gasdurchtrittsöffnungen 22. Die Fasem 19 der Lage 17 sind vorzugsweise im wesentlichen senkrecht oder in einem Winkel bezüglich der Austrittsseite 21 angeordnet, sodass eine lockere, kompressible dreidimensionale Struktur gebildet ist. Figur 9 zeigt einen Ausschnitt der Lage 17 in vergrössertem Massstab. Vorzugsweise besteht die Lage 17 aus ineinander verschlungenen Maschen.

Die Rückseite 33 des Applikators 11f ist gebildet durch ein durchlässiges Gewebe oder Gewirke 59, welches mindestens am Umfang mit dem Gewebe oder Gewirke 57 z.B. durch eine Naht 61, verbunden ist. Das Gewebe oder Gewirke 59 ist mit einer gasundurchlässigen Schicht 35, z.B. einer Kunststofffolie, abgedeckt. Die Gewebe oder Gewirke 57,59 können aus dem gleichen Material hergestellt und gleich ausgebildet sein.

Auf der Lage 17 sind in Abstand voneinander, flexible Kunststoffschläuche 13 angeordnet. Zwischen den Schläuchen 13 ist ein vliesartiges Material, Gewirke oder Gewebe 61 eingebracht, welches vorzugsweise ebenfalls gasdurchlässig ist. Das Material 61 kann wie die Lage 17 aus einer lockeren Faseranordnung bestehen. Die Schläuche 13 sind vom Gewebe oder Gewirke 59 überdeckt und mittels zwischen den Schläuchen vorgesehenen Längsnähten 63 in ihrer Lage fixiert.

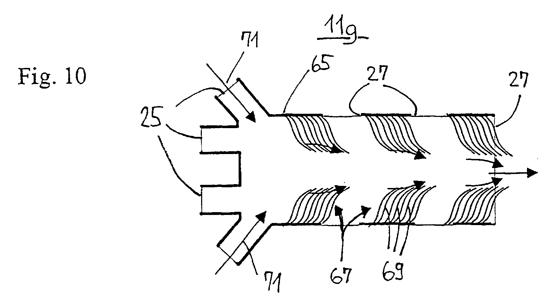

Figur 10 zeigt schematisch eine nicht zur Erfindung gehörende rohrförmige Ausführungsform 11g eines Applikators. Der Applikator 11g besitzt ein Rohr 65 mit mehreren Eintrittsöffnungen 25 und mehreren Austrittsöffnungen 27. Durch die stirnseitige, den Eintrittsöffnungen 25 gegenüberliegende Gasaustrittsöffnung 27 ist eine Extremität, z.B. ein Arm, in den Applikator 11g einführbar. An der Rohrinnenfläche ist ein Bürstenansatz 67 aus senkrecht oder in einem Winkel von der Rohrinnenfläche abstehenden, vorzugsweise elastischen Borsten 69 vorgesehen. Im Betrieb liegen die Borsten 69 an der Hautoberfläche an, und das durch die Eintrittsöffnungen 25 einströmende Gas (Pfeil 71) wird durch die Borsten 69 fein verteilt. Der Borstenansatz kann die gesamte oder nur einen Teil der Rohrinnenfläche bedecken.

Der Applikator resp. das Applikatorsystem ist einsetzbar für die kontrollierte und gezielte - flächen und/oder volumenbegrenzbaren Verteilung und/oder Dosierung von Kaltluft oder Kälte und/oder Warmluft oder Wärme - bei allen Erkrankungen und Zuständen, die direkt und indirekt mit Spastik, Ödemen, zirkulatorischen Problemen sowie Veränderungen von Spannungs, Längen-, Struktur-, biomechanischen-, neurogenen, stoffwechselmässigen und viskösen Charakteristika im zellulären, geweblichen, organischen, genetischen und physiologischen Bereich, insbesondere der Muskulatur, der Sehnen, der Knochen, des Gefäss- und Nervensystems von/bei Lebewesen zu tun haben.

Die Anwendung führt zu einer Effektivierung und Leistungssteigerung des neuromuskulären Systems und des Gefässsystems, sowie zu einer günstigen Beeinflussung und/oder Verbesserung der biomechanischen, neurophysiologischen und stoffwechselmässigen Qualitäten der Muskulatur und Sehnen, des gesamten Stütz- und Bewegungsapparates einschliesslich aller sonstigen Gewebsstrukturen. Es werden hormonelle, endokrine, zirkulatorische und mikrozirkulatorischen Aspekte des Körpers bezw. Körperareals günstig beeinflusst. Auch werden die Kriterien zur Verhinderung bezw. Beseitigung von Ödemen positiv beeinflusst. Es fördert hämodynamische, visköse und zirkulatorische Aspekte der Körperflüssigkeiten.

Die oben beschriebenen Applikatoren werden wie folgt verwendet: Flächige oder rohrförmige Applikatoren werden auf eine zu behandelnde Körperstelle aufgesetzt und anschliessend ein erwärmtes oder gekühltes gasförmiges Medium durchgeleitet. Das durch ein Kühl- oder Wärmegerät gekühlte oder erwärmte Medium gelangt über Verbindungsleitungen in die im oder am Applikator vorgesehenen Gasverteilräume. Durch die an den Gasverteilräumen vorgesehenen Austrittsöffnungen gelangt das Medium in die dreidimensionale Struktur, welche im wesentlichen nach allen Seiten, insbesondere auch parallel zur Gasdurchtrittsseite gasdurchlässig ist. Durch die dreidimensionale Struktur wird das austretende Medium noch im Applikator resp. der 3-D Struktur fein verteilt und gelangt dann durch die Durchtrittsöffnungen der Durchtrittsseite an die Umgebung resp. zur zu behandelnden Stelle.

Das gasförmige Medium kann selektiv durch ausgewählte oder alle Eintrittsöffnungen zugeführt werden. Denkbar ist, das Medium zu zirkulieren und die Temperatur des Medium mittels eines Heiz- resp. Kühlregelkreises zu regulieren. Dabei kann kaltes und/oder warmes Medium unter allen möglichen Durchführungsmodi wechselseitig, abwechselnd, zeitversetzt oder zeitgleich über die zu behandelnde Körperstelle und/ oder verschiedene Körperstellen geleitet werden. Dies erlaubt es, einen (Muskel-) Agonisten beispielsweise zu kühlen und den (Muskel-)Antagonisten zu erwärmen. Synergistische bzw. antagonistische Muskelketten können bedarfsgerecht mit Kälte oder Wärme behandelt werden. Insbesondere können mit dem Applikator zwei oder mehr voneinander und von der Umgebung getrennte Räume gebildet und getrennte Medienströme nach beliebigen Zeitmodi durch diese geleitet werden. Zweckmässigerweise werden die Menge sowie die Temperaturen des zugeführten und des abgeleiteten Mediums gemessen und gegebenenfalls die abgegebene Wärmemenge berechnet. Dies hat den Vorteil, dass die Behandlungen gut kontrollier- und reproduzierbar sind. Es ist insbesondere denkbar, die Oberflächentemperatur des Körpers und gegebenenfalls der im Applikator herrschende Druck mit je einem Sensor zu messen und die Temperatur und Druck des gasförmigen Mediums entsprechend einzustellen. Es kann ein Regelkreis vorgesehen sein, um eine wirkungsvolle Behandlung zu ermöglichen. Durch die oben beschriebenen Massnahmen können die Qualität und Quantität des Mediums sowie ihre Wirkung auf den Körper erfasst werden.

Die Verwendung der neuartigen Applikatoren erlaubt die Erzielung einer Wirkkopplung bzw. Synergie durch gleichzeitige Applikation aktiver und passiver Anwendungen wie z.B. Bewegung, physikalischer Therapie, pharmakologischer Präparate. Die Applikatoren können nach den speziellen Anforderungen an den Körper, Thermodynamik, Anwendungsabsicht, angepasst werden. Sie sind mit den verschiedensten Mess-, Steuer- und Regeleinrichtungen zur effektiven Anwendung und Applikation der Medien verwend- und kombinierbar.

Ein Applikator 11a-g für die oberflächliche Applikation eines gasförmigen Mediums auf einen Körperteil des lebenden menschlichen oder tierischen Organismus besitzt eine dreidimensionale Struktur mit einer Gasdurchtrittsseite 21 mit einer Vielzahl von Durchtrittsöffnungen 22 und einer der Gasdurchtrittsseite 21 gegenüberliegende Rückseite 33. Zwischen den Seiten 21,33 erstreckt sich die dreidimensionale Struktur, welche nach allen Seiten gasdurchlässig ist. Die Gasdurchtrittsseite 21 ist bei der Anwendung zum behandelnden Körperteil orientiert oder liegt an diesem vorzugsweise an. Des weiteren ist eine Einrichtung 13 zum Zuführen eines Gases oder Gasgemisches zur oder in die dreidimensionale Struktur vorgesehen. Die Zuführungsmittel sind so gestaltet ist, dass wenigstens ein Teil des im Betrieb zugeführten Gases durch oder zu den Durchtrittsöffnungen 22 der Gasdurchtrittsseite 21 strömt. Die dreidimensionale Struktur 15 bewirkt eine Gasfeinverteilung parallel zur Gasdurchtrittsseite 21, sodass das durch die Gasdurchtrittsseite 21 ausströmende Medium eine gleichmässige Temperatur- und Volumenverteilung besitzt. Die dreidimenstionale, der Gasverteilung dienende Struktur besitzt vorzugsweise eine Dicke von wenigstens 3 mm, vorzugsweise zwischen 3 und 30 mm, und ganz besonders bevorzugt zwischen 5 und 20 mm.
- 11: Applikator
- 13: Gaszuführungsmittel, z.B. Schläuche, Kissen
- 14: Gasdurchtritts- und Verteilungsmittel
- 15: dreidimensionale Struktur, z.B. textiles Abstandsgewebe oder -gewirke
- 17: aus Fäden und/oder Fasern bestehende Lage der dreidimensionalen Struktur
- 19: Fäden oder Fasern der dreidimensionalen Struktur
- 21: Gasdurchtrittsseite der dreidimensionalen Struktur
- 22: Gasdurchtrittsöffnungen der Gasdurchtrittsseite der 3-D Struktur
- 23: Kissen
- 25: Eintrittsöffnung der Gaszuführungs- und -verteilmittel, z.B. Schläuche
- 27: Austrittsöffnungen der Gaszuführungs- und -verteilmittel, z.B. Schläuche
- 29: Stopfen
- 31: regulierbares Ventil
- 33: körperabgewandte oder (=Rückseite) 33 des Applikators
- 35: gasundurchlässige Schicht
- 37: Seitenkanten
- 39: untere Lage der mehrlagigen Ausführung 11c
- 41: Bahnen der oberen Lage 43
- 43: obere Lage
- 45: innere Lage aus Schläuchen
- 47: Lagen aus einem dreidimensionalen Gewebe, Gewirke oder Vlies
- 49: Öffnung des Aplikators 11e
- 51: Distanzhalter
- 53: Applikatormanschette
- 57,59: Gewebe oder Gewirke
- 61: Naht am äusseren Rand
- 62: vliesartiges Material, Gewirke oder Gewebe
- 63: Längsnähte parallel zu den Schläuchen
- 65: Rohr
- 67: Bürstenansatz
- 69: Borsten
- 71: Richtung des im Betrieb durchströmenden, gasförmigen Mediums

## Patentansprüche

1. Applikator (11a-g) für die Applikation eines gekühlten oder erwärmten gasförmigen Mediums auf einen Körperteil des lebenden menschlichen oder tierischen Organismus mit
einem flexiblen, flächenhaft ausgebildeten, eine Vielzahl von Durchtrittsöffnungen (22) definierenden Gasdurchtrittsmittel (14) mit einer ersten eine
Gasdurchtrittsseite (21) definierenden Seite, welche bei der Anwendung zum behandelnden Körperteil orientiert ist,
einer Einrichtung (13) zum Zuführen eines Gases oder Gasgemisches zum oder an das Gasdurchtrittsmittel (14) mit
-mindestens einem Verteilraum (13), welcher sich im Bereich des flächenhaft ausgebildeten Gasdurchtrittsmittels erstreckt und eine Vielzahl von Austrittsöffnungen (27) besitzt, welche in Abstand zur Gasdurchtrittsseite (21) angeordnet sind,
**dadurch gekennzeichnet,**
**dass** das Gasdurchtrittsmittel (14) ein dreidimensionales, gasdurchlässiges aus Fasern oder Fäden gebildetes Abstandsgewebe oder Abstandsgewirke (15) einer ersten Art ist, welches eine Vielzahl von nach allen Seiten orientierten Gasdurchtrittsöffnungen oder Gasdurchtrittskanälen definiert, sodass im Betrieb eine Gasverteilung sowohl parallel als auch senkrecht zur Gasdurchtrittsfläche bewirkt ist.

2. Applikator nach Anspruch 1, **dadurch gekennzeichnet, dass** die Fasern oder Fäden (19) im wesentlichen senkrecht oder in einem Winkel bezüglich der Gasaustrittsseite (21) angeordnet sind.

3. Applikator nach Anspruch 1 oder 2, **dadurch gekennzeichnet, dass** die Gasdurchtrittsseite (21) aus einem Gewebe oder Gewirke (57,59) einer zweiten Art besteht, welches mit dem dreidimensionalen Abstandsgewirke (15) verbunden ist.

4. Applikator nach einem der Ansprüche 1 bis 3, **dadurch gekennzeichnet, dass** das dreidimensionale Abstandsgewirke (15) mindestens eine Dicke von 3 mm und vorzugsweise zwischen 3 und 30 mm hat.

5. Applikator nach Anspruch 1, **dadurch gekennzeichnet, dass** das dreidimensionale Abstandsgewebe oder -gewirke (15) aus einem Feuchtigkeit aufnehmenden und/ oder ableitenden Material hergestellt ist.

6. Applikator nach einem der Ansprüche 1 bis 5, **dadurch gekennzeichnet, dass** eine Mehrzahl von in Abstand voneinander angeordneten Verteilräumen (13) vorgesehen sind.

7. Applikator nach einem der Ansprüche 1 bis 6, **dadurch gekennzeichnet, dass** der Verteilraum (13) ein Schlauch, ein Rohr, ein Sack oder dergleichen ist.

8. Applikator nach einem der Ansprüche 1 bis 7, **dadurch gekennzeichnet, dass** die Austrittsöffnungen (27) wenigstens in Richtung der Durchtrittsseite (23) orientiert sind.

9. Applikator nach einem der Ansprüche 1 bis 8, **dadurch gekennzeichnet, dass** der Applikator (11a-g) flexibel ausgebildet ist, sodass der Applikator (11a-g) an unterschiedliche Körperpartien anpassbar resp. anlegbar ist.

10. Applikator nach einem der Ansprüche 1 bis 9, **dadurch gekennzeichnet, dass** die Gaszuführungseinrichtung (13) in oder an der dreidimensionalen Struktur (15) angeordnet ist.

11. Applikator nach einem der Ansprüche 1 bis 10, **dadurch gekennzeichnet, dass** der Applikator (11a-g) eine zweite, vorzugsweise gasundurchlässige Seite hat, welche der ersten Gasdurchtrittsseite (21) gegenüberliegt, und dass die Gasverteilräume (13) vorzugsweise zwischen der ersten und zweiten Seite angeordnet sind.

12. Applikator nach einem der Ansprüche 1 bis 11, **dadurch gekennzeichnet, dass** die dreidimensionale Struktur (15) mehrere Lagen (39,43) aufweist.

13. Applikator nach einem der Ansprüche 1 bis 12, **dadurch gekennzeichnet, dass** am Verteilraum (13) Leitflächen für die Direktion des während des Betriebs austretenden Gasmediums in Richtung der Durchtrittsseite (23) vorgesehen sind.

14. Applikator nach einem der Ansprüche 1 bis 13, **dadurch gekennzeichnet, dass** am Applikator (11a-g) Befestigungsmittel zum Festmachen des Applikators (11a-g) an oder über der zu behandelnden Körperpartie vorgesehen sind.

15. Applikator nach Anspruch 14, **dadurch gekennzeichnet, dass** die Befestigungsmittel, z.B. mindestens ein Band, ein Gurt oder dergleichen, integral mit dem Applikator (11a-g) sind.

16. Applikator nach einem der Ansprüche 1 bis 15, **dadurch gekennzeichnet, dass** der Applikator (11a-g) im wesentlichen flach, z.B. in Gestalt eines Kissens (11f), ausgebildet ist.

17. Applikator nach einem der Ansprüche 1 bis 16, **dadurch gekennzeichnet, dass** der Applikator die Gestalt einer über eine Körperextremität überstülpbaren Manschette (53) hat.

18. Applikator nach einem der Ansprüche 1 bis 17, **dadurch gekennzeichnet, dass** der Applikator (11a-g) mindestens ein Fenster im Gasdurchtrittsmittel (14) aufweist.

19. Applikatorsystem mit wenigstens einem Kälte- und/oder Wärmegerät zur Erzeugung eines gekühlten oder erwärmten gasförmigen Mediums und einem Applikator (11a-g) gemäss einem der Ansprüche 1 bis 18 sowie gegebenenfalls Verbindungsmitteln zum Verbinden des Kälte-/Wärmegeräts mit dem Applikator (11a-g).

## Claims

1. An applicator (11a-g) for the application of a cooled or warmed gaseous medium to a part of the body of the living human or animal organism with
a flexible gas passage means (14) designed as a flat body and defining a plurality of through openings (22) with a first side defining a gas passage side that faces the part of the body to be treated when the applicator is in use, and
a device (13) for supplying a gas or gas mixture to or onto the gas passage means (14) with at least one distribution space that extends in the region of the gas passage means and is provided with a plurality of gas outlets openings (27), which are arranged at a certain distance from the gas passage side (21),
**characterized in that**
the gas passage means (14) is a three-dimensional woven or knitted spacer fabric (15) of a first kind that is permeable for gases and is made of fibres or yams, the fabric defining a plurality of gas passage openings or gas passage channels oriented in all directions, so that gas distribution is assured both parallel to the gas passage surface and ar right angles thereto during operation.

2. An applicator in accordance with Claim 1, **characterized in that** the fibres or yams are arranged at an essentially right angle or at an angle relative to the gas passage side (21).

3. An applicator in accordance with Claim 1 or 2, **characterized in that** the gas passage side (21) consists of a woven or knitted spacer fabric (57,59) of a second kind, which is connected to the threedimensional woven or knitted spacer fabric (15).

4. An applicator in accordance with any of Claims 1 to 3, **characterized in that** the threedimensional woven or knitted spacer fabric (15) has a thickness of at least 3 mm and preferably between 3 and 30 mm.

5. An applicator in accordance with Claim 1, **characterized in that** the three-dimensional woven or knitted spacer fabric is made of a material that absorbs and/or removes moisture.

6. An applicator in accordance with any one of Claims 1 to 5 **characterized in that** there are provided several distribution spaces (13) arranged at a certain distance from each other.

7. An applicator in accordance with any one of Claims 1 to 6, **characterized in that** the distribution space (13) is a hose, a tube, a bag or something similar.

8. An applicator in accordance with any one of Claims 1 to 7, **characterized in that** the outlet openings (27) are oriented at least in the direction of the gas passage side (23).

9. An applicator in accordance with any one of Claims 1 to 8, **characterized in that** the applicator (11a-g) is designed to be flexible, so that the applicator (11a-g) may be adapted to and/or be placed against different parts of the body.

10. An applicator in accordance with any one of Claims 1 to 9, **characterized in that** the gas supply device (13) is arranged in or on the three-dimensional structure (15).

11. An applicator in accordance with any one of Claims 1 to 10, **characterized in that** the applicator (11a-g) has a second side, preferably impermeable for gases, lying opposite the first gas passage side (21), and that the gas distribution spaces (13) are preferably arranged between the first and the second side.

12. An applicator in accordance with any one of Claims 1 to 11, **characterized in that** the three-dimensional structure (15) consists of several layers (39, 43).

13. An applicator in accordance with any one of Claims 1 to 12, **characterized in that** the distribution space (13) is provided with guide surfaces to orient the gas medium issuing during operation of the applicator in direction of the gas passage side (23).

14. An applicator in accordance with any one of Claims 1 to 13, **characterized in that** the applicator (11a-g) is provided with fixing means for attaching the applicator (11a-g) to or above the part of the body that is to be treated.

15. An applicator in accordance with Claim 14, **characterized in that** the fixing means, for example a strap, a belt or similar, are integral with the applicator (11a-g).

16. An applicator in accordance with any one of Claims 1 to 15, **characterized in that** the applicator (11a-g) is essentially designed as a flat body, in the form of a cushion (11f) for example.

17. An applicator in accordance with any one of Claims 1 to 16, **characterized in that** the applicator has the form of a sleeve (53) that can be placed over an extremity of the body.

18. An applicator in accordance with any one of Claims 1 to 17, **characterized in that** the applicator (11a-g) is provided with at least one window in the gas passage means (14).

19. An applicator system with at least one cooling and/or heating device for the production of a cooled or heated gaseous medium and an applicator (11a-g) in accordance with any one of Claims 1 to 18, as well as, whenever necessary, connection means to connect the cooling/heating device to the applicator (11a-g).

## Revendications

1. Applicateur (11 a-g) pour l'application d'un agent gazeux refroidi ou réchauffé sur une partie du corps de l'organisme humain ou animal vivant avec
un moyen de passage du gaz (14) flexible, configuré en surface plane, qui définit une multitude d'ouvertures de passage (22) avec un premier côté qui définit un côté de passage du gaz (21) qui est orienté, lors de l'aplication, vers la partie du corps à traiter,
un dispositif (13) pour amener un gaz ou un mélange de gaz vers ou au moyen de passage du gaz (14) avec
au moins un espace de répartition (13) qui s'étend dans la zone du moyen de passage du gaz configuré en surface plane et qui possède une multitude d'ouvertures de sortie (27) qui sont placées à un écartement du côté de passage du gaz (21),
**caractérisé en ce**
**que** le moyen de passage du gaz (14) est un tissu d'écartement ou un tricot d'écartement (15) d'un premier type, tridimensionnel, perméable au gaz, formé en fibres ou fils, tissu qui définit une multitude d'ouvertures de passage du gaz ou de canaux de passage du gaz orientés vers tous les côtés si bien qu'en service il est provoqué une répartition du gaz aussi bien parallèlement que perpendiculairement à la surface de passage du gaz.

2. Applicateur selon la revendication 1, **caractérisé en ce que** les fibres ou fils (19) sont placés substantiellement perpendiculairement ou en formant un angle avec le côté de passage du gaz (21).

3. Applicateur selon la revendication 1 ou 2, **caractérisé en ce que** le côté de passage du gaz (21) est constitué par un tissu ou un tricot (57, 59) d'un second type qui est relié au tricot d'écartement tridimensionnel (15).

4. Applicateur selon l'une des revendications 1 à 3, **caractérisé en ce que** le tricot d'écartement tridimensionnel (15) a au moins une épaisseur de 3 mm et de préférence entre 3 et 30 mm.

5. Applicateur selon la revendication 1, **caractérisé en ce que** le tissu ou tricot d'écartement tridimensionnel (15) est fabriqué en une matière qui absorbe et/ou évacue l'humidité.

6. Applicateur selon l'une des revendications 1 à 5, **caractérisé en ce qu'**il est prévu une multitude d'espaces de répartition (13) écartés l'un de l'autre.

7. Applicateur selon l'une des revendications 1 à 6, **caractérisé en ce que** l'espace de répartition (13) est un tuyau, un tube, un sac ou équivalent.

8. Applicateur selon l'une des revendications 1 à 7, **caractérisé en ce que** les ouvertures de sortie (27) sont orientées au moins en direction du côté de passage (23).

9. Applicateur selon l'une des revendications 1 à 8, **caractérisé en ce que** l'applicateur (11 a-g) est configuré flexible si bien que l'applicateur (11 a-g) peut être adapté ou appliqué à différentes parties du corps.

10. Applicateur selon l'une des revendications 1 à 9, **caractérisé en ce que** le dispositif d'alimentation de gaz (13) est placé dans ou sur la structure tridimensionnelle (15).

11. Applicateur selon l'une des revendications 1 à 10, **caractérisé en ce que** l'applicateur (11 a-g) a un second côté, de préférence imperméable au gaz, qui est en face du premier côté de passage du gaz (21) et que les espaces de distribution du gaz (13) sont placés de préférence entre le premier et le second côté.

12. Applicateur selon l'une des revendications 1 à 11, **caractérisé en ce que** la structure tridimensionnelle (15) présente plusieurs couches (39, 43).

13. Applicateur selon l'une des revendications 1 à 12, **caractérisé en ce que** des surfaces conductrices pour la direction de l'agent gazeux qui sort pendant le fonctionnement en direction du côté de passage (23).

14. Applicateur selon l'une des revendications 1 à 13, **caractérisé en ce que** des moyens de fixation sont prévus pour fixer l'applicateur (11 a-g) à ou au-dessus de la partie du corps à traiter.

15. Applicateur selon la revendication 14, **caractérisé en ce que** les moyens de fixation, par exemple au moins une bande, une courroie ou équivalent, font un bloc avec l'applicateur (11 a-g).

16. Applicateur selon l'une des revendications 1 à 15, **caractérisé en ce que** l'applicateur (11 a-g) est configuré substantiellement plat, par exemple en forme de coussin (11f).

17. Applicateur selon l'une des revendications 1 à 16, **caractérisé en ce que** l'applicateur a la configuration d'une manchette (53) qui peut être passée sur une extrémité du corps.

18. Applicateur selon l'une des revendications 1 à 17, **caractérisé en ce que** l'applicateur (11 a-g) présente au moins une fenêtre dans le moyen de passage du gaz (14).

19. Système applicateur avec au moins un appareil de refroidissement et/ou de chauffage pour produire un agent gazeux refroidi ou réchauffé et un applicateur (11 a-g) conformément à l'une des revendications 1 à 18 ainsi qu'éventuellement des moyens de liaison pour relier l'appareil de refroidissement/de chauffage à l'applicateur (11 a-g).
